# EUROPÄISCHE PATENTANMELDUNG

(11) **EP 3 199 204 A1**
(43) Veröffentlichungstag der Anmeldung: **02.08.2017**
(21) Anmeldenummer: 16153359.1
(22) Anmeldetag: 29.01.2016
(51) Int. Cl.: A61Q 5/02, A61Q 9/02, A61Q 11/00, A61Q 19/10, A61K 8/24, A61K 8/365, A61K 8/46, C11D 1/12, C11D 3/04, C11D 3/06, C11D 3/20

(54) **WÄSSRIGE TENSID-ZUSAMMENSETZUNGEN**

(71) Anmelder: BASF SE, 67056 Ludwigshafen am Rhein (DE)
(72) Erfinder: Behler, Ansgar, 46240 Bottrop (DE); Clasen, Frank, 40723 Hilden (DE); Brunn, Claudia, 40597 Duesseldorf (DE); Pottie, Laurence, 50823 Koeln (DE); Barbenheim, Monika, 46240 Bottrop (DE); Hoff, Daniel, 41517 Grevenbroich (DE)
(74) Vertreter: Reinhardt, Jürgen

(57) **Zusammenfassung**

Vorgeschlagen werden Zusammensetzungen enthaltend
• ein oder mehrere **Alkyllactatsulfate (A)** der allgemeinen Formel (I),

R¹(OCOCH(CH₃))ₙOSO₃M¹ (I)

worin der Rest R¹ einen linearen oder verzweigten Alkyl- oder Alkenylrest mit 6 bis 30 C-Atomen bedeutet, der Index n eine Zahl im Bereich von 1 bis 5 ist, und der Rest M¹ ausgewählt wird aus der Gruppe H, Li, Na, K, Ca/2, Mg/2, Ammonium und Alkanolamin,
• ein oder mehrere **Verbindungen (B)**, die ausgewählt werden aus der Gruppe Phosphorsäure und deren Mono-, Di-, und Trisalzen, wobei das Kation dieser Salze ausgewählt wird aus der Gruppe Li, Na, K, Ca, Mg, Ammonium und Alkanolamin,
• ein oder mehrere **Verbindungen (C)**, die ausgewählt werden aus der Gruppe Citronensäure und deren Mono-, Di-, und Trisalzen, wobei das Kation dieser Salze ausgewählt wird aus der Gruppe Li, Na, K, Ca, Mg, Ammonium und Alkanolamin, und
• **Wasser**
wobei folgende Maßgaben gelten:
• Das Gewichtsverhältnis der Summe der Verbindungen (B) und (C) zur Summe der Verbindungen (A) liegt im Bereich von 1 : 5 bis 1 : 10.
• Das Gewichtsverhältnis der Summe der Verbindungen (B) zur Summe der Verbindungen (C) liegt im Bereich von 1 : 1,2 bis 1 : 3.
• Der pH-Wert der Zusammensetzungen liegt im Bereich von 4,5 bis 7,5.

## Beschreibung

### Gebiet der Erfindung

Die vorliegende Erfindung betrifft lagerstabile wäßrige Zusammensetzungen mit einem Gehalt an ein oder mehreren Alkyllactatsulfaten.

### Stand der Technik

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzend auch als Ethersulfate bezeichnet). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

DE 40,03,096 A1 beschreibt sulfatierte Hxdrocycarbonsäureester und deren Verwendung als oberflächenaktive Substanzen.

EP 2,810,935 A1 beschreibt sulfatierte Ester von Oligohydroxycarbonsäuren und deren Verwendung als oberflächenaktive Substanzen.

### Beschreibung der Erfindung

Die oben genannte EP 2,810,935 A1 beschreibt sulfatierte Ester von Oligohydroxycarbonsäuren als oberflächenaktive Substanzen (Tenside), wobei diese Verbindungen wegen der Estergruppe eine gewisse Hydrolyse-Neigung zeigen, die sich negativ auf die Lagerstabilität auswirkt. Die Aufgabe der vorliegenden Erfindung hat darin bestanden, wässrige Tensid-Zusammensetzungen bereitzustellen, die Verbindungen der in der EP 2,810,935 A1 mittels der dort angegebenen Formel (I) beschriebenen Art enthalten, bei denen der Rest R³ eine Methylgruppe bedeutet und wobei der Index n neben der dort angegebenen Bedeutung auch null sein kann, und die eine für praktische Zwecke ausreichende Lagerstabilität aufweisen.

Unter Lagerstabilität wird im Rahmen der vorliegenden Anmeldung verstanden, dass sich der pH-Wert der erfindungsgemäßen Zusammensetzungen bei 90-tägiger Lagerung bei 40 °C um 1,2 oder weniger nach unten hin, d.h. in Richtung geringerer pH-Werte, ändert. Beispiel: Bei einem Start-pH-Wert von 5,9 darf der pH-Wert der Zusammensetzung bei 90-tägiger Lagerung bei 40 °C maximal auf 4,7 sinken.

Gegenstand der Erfindung sind zunächst Zusammensetzungen enthaltend
- ein oder mehrere **Alkyllactatsulfate (A)** der allgemeinen Formel (I),

   R¹(OCOCH(CH₃))ₙOSO₃M¹ (I)

   worin der Rest R¹ einen linearen oder verzweigten Alkyl- oder Alkenylrest mit 6 bis 30 C-Atomen bedeutet, der Index n eine Zahl im Bereich von 1 bis 5 ist, und der Rest M¹ ausgewählt wird aus der Gruppe H, Li, Na, K, Ca/2, Mg/2, Ammonium und Alkanolamin,
- ein oder mehrere **Verbindungen (B),** die ausgewählt werden aus der Gruppe Phosphorsäure und deren Mono-, Di-, und Trisalzen, wobei das Kation dieser Salze ausgewählt wird aus der Gruppe Li, Na, K, Ca, Mg, Ammonium und Alkanolamin,
- ein oder mehrere **Verbindungen (C),** die ausgewählt werden aus der Gruppe Citronensäure und deren Mono-, Di-, und Trisalzen, wobei das Kation dieser Salze ausgewählt wird aus der Gruppe Li, Na, K, Ca, Mg, Ammonium und Alkanolamin, und
- **Wasser,**
wobei folgende Maßgaben gelten:
- Das Gewichtsverhältnis der Summe der Verbindungen (B) und (C) zu den Verbindungen (A) liegt im Bereich von 1 : 5 bis 1 : 10;
- das Gewichtsverhältnis der Summe der Verbindungen (B) zur Summe der Verbindungen (C) liegt im Bereich von 1 : 1,2 bis 1 : 3; und
- der pH-Wert der Zusammensetzungen liegt im Bereich von 4,5 bis 7,5.

### Zu den Verbindungen (A)

Die Verbindungen (A), die hier als **Alkyllactatsulfate** bezeichnet werden, sind für die erfindungsgemäßen wässrigen Tensid-Zusammensetzungen obligatorisch. Sie haben die oben angegebene Formel (I)

R¹(OCOCH(CH₃))ₙOSO₃M¹ (I)

worin der Rest R¹ einen linearen oder verzweigten Alkyl- oder Alkenylrest mit 6 bis 30 C-Atomen bedeutet, der Index n eine Zahl im Bereich von 1 bis 5 ist, und der Rest M¹ ausgewählt wird aus der Gruppe H, Li, Na, K, Ca/2, Mg/2, Ammonium und Alkanolamin.

Ausdrücklich sei festgestellt, dass die Bezeichnung der Verbindungen (A) als **Alkyllactatsulfa**te lediglich einer sprachlich einfachen Bezeichnung der Verbindungen (A) dient und nicht strukturell einschränkend zu verstehen ist; denn in der formelmäßigen Definition der Verbindungen (A) ist klargestellt, dass der Rest R¹ sowohl einen Alkyl- als auch einen Alkenylrest bedeuten kann und ferner - dies zeigt der Index n - dass es sich um Alkyl- bzw. Alkenyllactatsulfate und um Alkyl- bzw. Alkenyloligolactatsulfate handeln kann.

Die Indexzahl n in der allgemeinen Formel (I) gibt den Oligomerisierungsgrad an. Der Oligomerisierungsgrad der Verbindungen (I) liegt zwischen 1 und 5 und vorzugsweise zwischen 1,05 und 2,0. Während n in einem einzelnen Molekül der Formel (I) stets ganzzahlig ist und hier vor allem die Werte im Bereich von 1 bis 5 annimmt, ist der Wert n für ein Alkyllactatsulfat, das eine Mischung verschiedener Moleküle (I) darstellt, die sich in den je individuellen n-Werten unterscheiden, eine analytisch ermittelte rechnerische Größe, die meistens eine gebrochene Zahl darstellt. Vorzugsweise werden Alkyloligolactatsulfate mit einem mittleren Oligomerisierungsgrad n im Bereich von 1,1 bis 1,5 eingesetzt.

Der Rest R¹ leitet sich vorzugsweise von gesättigten, linearen, primären Alkoholen mit 10 bis 18 C-Atomen und vorzugsweise 12 bis 16 C-Atomen und insbesondere 12 bis 14 C-Atomen ab. Typische Beispiele für geeignete Reste R¹ sind Octyl-, Decyl-, Undecyl-, Dodecyl- und Myristyl-. Sie leiten sich von den gesättigten Fettalkoholen Caprylalkohol (Octanol-1), Caprinalkohol (Decanol-1), Undecanol-1, Laurylalkohol (Dodecanol-1) und Myristylalkohol (Tetradecanol-1) ab, wie sie beispielsweise bei der Hydrierung von technischen Fettsäuremethylestern oder im Verlauf der Hydrierung von Aldehyden bei der Roelen'schen Oxosynthese erhalten werden.

Der Rest M¹ der Verbindungen (I) wird wie oben ausgeführt ausgewählt aus der Gruppe H, Li, Na, K, Ca/2, Mg/2, Ammonium und Alkanolamin. Als Alkanolamine besonders bevorzugt sind dabei Monoethanolamin, Diethanolamin, Triethanolamin und Mono-Isopropanolamin.

In einer besonders bevorzugten Ausführungsform der Verbindungen (I) hat der Rest M¹ die Bedeutung Natrium (Na) und/oder Kalium (K).

Die Verbindungen (A) können nach allen dem Fachmann einschlägig bekannten Methoden hergestellt werden. Eine besonders bevorzugte Methode der Herstellung ist dabei die Umsetzung von Alkoholen R¹OH, worin R¹ die oben genannte Bedeutung hat, mit der entsprechenden Menge an Milchsäure CH₃-CH(OH)-COOH, der in der IUPAC-Nomenklatur die Bezeichnung 2-Hydroxypropansäure zukommt, nebst anschließender Sulfierung mit gasförmigem Schwefeltrioxid sowie gewünschtenfalls Neutralisation der sauren Sulfierprodukte.

In einer bevorzugten Ausführungsform bedeutet der Rest R¹ in den Verbindungen (A) einen linearen, gesättigten Alkylrest mit 10 bis 18 C-Atomen, insbesondere ist Vorzugsweise bedeutet der Rest R¹ in den Verbindungen (A) einen linearen, gesättigten Alkylrest mit 12 bis 16 C-Atomen, wobei insbesondere Mischungen von verbindungen (A) bevorzugt sind, bei denen der Rest R¹ zu etwa 70 Gew.-% ein Laurylrest und zu 30 Gew.-% ein Myristylrest ist.

### Zur den Verbindungen (B)

Die Verbindungen (B), werden ausgewählt aus der Gruppe Phosphorsäure und deren Mono-, Di-, und Trisalzen, wobei das Kation dieser Salze ausgewählt wird aus der Gruppe Li, Na, K, Ca, Mg, Ammonium und Alkanolamin. Als Alkanolamine besonders bevorzugt sind dabei Monoethanolamin, Diethanolamin, Triethanolamin und Mono-Isopropanolamin. In einer besonders bevorzugten Ausführungsform der Verbindungen (B) handelt es sich bei dem Kation um Natrium (Na) und/oder Kalium (K).

In der Regel werden, abhängig vom pH-Wert der Zusammensetzungen - und in diesem Zusammenhang sei ausdrücklich darauf hingewiesen, dass der pH-Wert der erfindungsgemäßen wässrigen Zusammensetzungen ein obligatorischer Parameter ist - Mischungen verschiedener Substanzen (B) vorliegen.

### Zur den Verbindungen (C)

Die Verbindungen (C), werden ausgewählt aus der Gruppe Citronensäure und deren Mono-, Di-, und Trisalzen, wobei das Kation dieser Salze ausgewählt wird aus der Gruppe Li, Na, K, Ca, Mg, Ammonium und Alkanolamin. Als Alkanolamine besonders bevorzugt sind dabei Monoethanolamin, Diethanolamin, Triethanolamin und Mono-Isopropanolamin. In einer besonders bevorzugten Ausführungsform der Verbindungen (B) handelt es sich bei dem Kation um Natrium (Na) und/oder Kalium (K).

In der Regel werden, abhängig vom pH-Wert der Zusammensetzungen - und in diesem Zusammenhang sei ausdrücklich darauf hingewiesen, dass der pH-Wert der erfindungsgemäßen wässrigen Zusammensetzungen ein obligatorischer Parameter ist - Mischungen verschiedener Substanzen (C) vorliegen.

### Bevorzugte Ausführungsformen

Wie oben ausgeführt, gelten für die erfindungsgemäßen Zusammensetzungen folgende Maßgaben:
- Das Gewichtsverhältnis der Summe der Verbindungen (B) und (C) zur Summe der Verbindungen (A) liegt im Bereich von 1 : 5 bis 1 : 10 und vorzugsweise im Bereich von 1 : 6 bis 1: 7,5.
- Das Gewichtsverhältnis der Summe der Verbindungen (B) zur Summe der Verbindungen (C) liegt im Bereich von 1 : 1,2 bis 1 : 3 und vorzugsweise im Bereich von 1 : 1,5 und 1 : 2,5.
- Der pH-Wert der Zusammensetzungen liegt im Bereich von 4,5 bis 7,5 und vorzugsweise im Bereich von 5,0 bis 7,0 und insbesondere im Bereich von 5,7 bis 6,7.

### Optionale Ausführungsformen

In einer Ausführungsform enthalten die erfindungsgemäßen wässrigen Tensid-Zusammensetzungen neben den Verbindungen (A), (B), (C) und Wasser zusätzlich ein oder mehrere Alkohole (D), Alkylsulfate (E), Alkyllactate (F), Milchsäure (G), Milchsäuresulfat (H), Schwefelsäuresalze (H) und anorganische Salze (S). Dabei gilt die Maßgabe, dass die Summe sämtlicher Verbindungen (D) bis (S) - angegeben als Gew.-%, bezogen auf die gesamte Zusammensetzung - kleiner ist als die Menge der Verbindungen (A) - ebenfalls angegeben als Gew.-%, bezogen auf die gesamte Zusammensetzung. In einer bevorzugten Ausführungsform beträgt dabei die Menge der Verbindungen (A) 5 bis 50 Gew.-% bezogen auf die gesamte Zusammensetzung - und insbesondere 8 bis 40 Gew.-%.

Diese Verbindungen (D) bis (H) werden nachfolgend beschrieben:

### Zur den Verbindungen (D)

Bei den Verbindungen (D) handelt es sich um Alkohole der Formel R¹-OH, wobei R¹ die oben für die Verbindungen (A) angegebene Bedeutung hat.

### Zur den Verbindungen (E)

Bei den Verbindungen (E) handelt es sich um Alkylsulfate der Formel R¹-OSO₃M¹, wobei R¹ und M¹ die oben für die Verbindungen (A) angegebene Bedeutung haben.

### Zur den Verbindungen (F)

Bei den Verbindungen (F) handelt es sich um Alkyllactate der Formel

R¹(OCOCH(CH₃))ₙOH

wobei R¹ und n die oben für die Verbindungen (A) angegebene Bedeutung haben.

### Zur den Verbindungen (G)

Bei den Verbindungen (F) handelt es sich um Milchsäure (2-Hydroxypropansäure), der bekanntlich die Formel HOCOCH(CH₃)OH zukommt.

### Zur den Verbindungen (H)

Bei den Verbindungen (H) handelt es sich um Milchsäuresulfat, der die Formel HOCOCH(CH₃)OSO₃M¹ zukommt, wobei M¹ die oben für die Verbindungen (A) angegebene Bedeutung hat.

### Zur den Verbindungen (S)

Bei den Verbindungen (S) handelt es sich um anorganische Salze der Schwefelsäure. Diese Salze haben die Formel (M¹)₂SO₄, wobei M¹ die oben für die Verbindungen (A) angegebene Bedeutung hat.

Gewünschtenfalls können die erfindungsgemäßen wässrigen Tensid-Zusammensetzungen zusätzlich ein oder mehrere weitere Tenside enthalten, die strukturell nicht zu den oben genannten Verbindungen (A) und (E) zählen. Bei diesen Tensiden kann es sich um anionische, kationische, nichtionische oder amphotere Tenside handeln.

### Verwendung der Zusammensetzungen

Ein weiterer Erfindungsgegenstand ist die Verwendung der oben genannten Zusammensetzungen für kosmetische Mittel, sowie Wasch- und Reinigungsmittel.

Im Hinblick auf kosmetische Mittel sind dabei insbesondere solche besonders bevorzugt, die in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten (etwa Zahnpasten, Mundwässern und dergleichen) vorliegen.

### Beispiele

### Eingesetzte Substanzen

**VE-Wasser** = vollentsalztes Wasser
**Alkyllactatsulfat ALS-I:** Wässrige Zusammensetzung, enthaltend eine Verbindung (A), wobei folgende Parameter gelten: n = 1,11 (Mittelwert der Spezies mit n= 1 und n=2); R¹ = 70% C₁₂ und 30% C₁₄; M¹ = Na, ferner die Verbindungen (D) bis (S). Analytik: Die Zusammensetzung enthält 9,4 Gew.-% (A) und in Summe 6,3 Gew.-% der Verbindungen (D) bis (S), Rest Wasser. Die Zusammensetzung stellt eine bei 20°C klare, homogene, leicht gelbliche Flüssigkeit dar.
**Alkyllactatsulfat ALS-II:** Wässrige Zusammensetzung, enthaltend eine Verbindung (A), wobei folgende Parameter gelten: n = 1,23 (Mittelwert der Spezies mit n= 1 und n=2); R¹ = 70% C₁₂ und 30% C₁₄; M¹ = Na, ferner die Verbindungen (D) bis (S). Analytik: Die Zusammensetzung enthält 10,9 Gew.-% (A) und in Summe 4,6 Gew.-% der Verbindungen (D) bis (S), Rest Wasser. Die Zusammensetzung stellt eine bei 20°C klare, homogene, leicht gelbliche Flüssigkeit dar.
**Kaliumdihydrogenphosphat: KH₂PO₄,** ≥99% p.a. (Chemikalienhandel)
**Natriumcitrat:** Trinatriumcitratdihydrat, ≥99% p.a. (Chemikalienhandel)

### Mess- und Prüfmethoden

**pH-Wert:** Unter Einsatz eines handelsüblichen pH-Meters, wurde der pH-Wert direkt in der Formulierung, also der wässrigen Tensid-Zusammensetzung, gemessen.
**Aktivsubstanzgehalt:** Die Bestimmung des Aniontensidgehalts erfolgte mittels Epton-Titration (gemäß ISO 2271), erfasst wird hierüber der Gehalt der Verbindungen (A) und (E).
**Aussehen:** Die zu prüfenden Muster wurden in 250ml Glasflaschen gelagert und visuell beurteilt hinsichtlich dem Auftreten von Trübungen, Inhomogenität (z.B. Ausfällungen, Schlieren, Bodensatz) und insbesondere Phasentrennung, da dies ein Indikator von Hydrolyse ist. Im Fall der eingesetzten Muster schwamm der durch Hydrolyse freiwerdende Fettalkohol oben auf.
**Lagertest:** Die Muster wurden in 250ml Glasflaschen in einem Wärmeschrank bei 40°C gelagert und in regelmäßigen Abständen entnommen, auf 20 °C abgekühlt und dann das Aussehen beurteilt. Sofern die Lösungen noch einphasig, klar und homogen waren, wurde der pH-Wert gemessen und die Muster in den Wärmeschrank zurückgestellt.

Bei der Lagerung des Musters gemäß Beispiel B2 wurde zusätzlich monatlich der Aniontensidgehalt bestimmt um nachzuweisen, dass Abnahme des pH-Werts und Aussehen des Musters mit Abbau des Aniontensidgehalts korrelieren.

### Beispiel B1:

Das Alkyllactatsulfat ALS-I wurde mit 0,5% Kaliumdihydrogenphosphat und 1,0% Natriumcitrat - Gew.-% jeweils bezogen auf die gesamte Zusammensetzung - versetzt und dann bei 20 °C gerührt bis alle Feststoffe gelöst waren und eine klare homogene Lösung vorlag. Der Start-pH-Wert der Lösung wurde gemessen. Danach wurde die Probe wie oben beschrieben gelagert. Die Messdaten sind den Tabellen 1 und 2 zu entnehmen.

### Vergleichsbeispiel VB1:

Analog wie Beispiel B1, jedoch ohne Zugabe von Puffersubstanzen. Die Messdaten sind den Tabellen 1 und 2 zu entnehmen.

### Vergleichsbeispiele VB2 bis VB4:

Analog wie Beispiel B1, jedoch unter Variation der Mengen an Kaliumdihydrogenphosphat und Natriumcitrat. Die Messdaten sind den Tabellen 1 und 2 zu entnehmen.

**Tabelle 1: Übersicht Beispiele/Vergleichsbeispiele**

| | Start-pH-Wert | KH₂PO₄ | Natriumcitrat |
|---|---|---|---|
| VB 1 | 6,7 | 0 Gew.-% | 0 Gew.-% |
| VB 2 | 6,2 | 0,5 Gew.-% | 0 Gew.-% |
| VB 3 | 6,1 | 0 Gew.-% | 0,5 Gew.-% |
| VB 4 | 5,7 | 0,5 Gew.-% | 0,5 Gew.-% |
| B 1 | 5,9 | 0,5 Gew.-% | 1,0 Gew.-% |

**Tabelle 2: pH-Wertänderung (Start-pH-Wert minus pH-Werte der Probe nach x Tagen) während Lagerung bei 40°C**

| | 7 Tage | 13 Tage | 20 Tage | 28 Tage | 41 Tage | 62 Tage | 81 Tage | 90 Tage | 125 Tage | 178 Tage |
|---|---|---|---|---|---|---|---|---|---|---|
| VB 1 | -1,9 | | -2,7 | X | | | | | | |
| VB 2 | -0,3 | -0,7 | -0,7 | -0,8 | -1,0 | -2,3 | X | | | |
| VB 3 | -0,6 | -0,9 | -0,9 | -1,0 | -1,1 | -1,6 | -2,3 | X | | |
| VB 4 | -0,3 | -0,7 | -0,6 | -0,7 | -0,8 | -1,3 | -2,0 | X | | |
| B 1 | -0,2 | -0,6 | -0,5 | -0,5 | -0,5 | -0,8 | -1,0 | -1,1 | -1,3 | X |

| | | | | | | | | | | |
|---|---|---|---|---|---|---|---|---|---|---|
| X = Probe war 2-phasig | | | | | | | | | | |

Gemäß Tabelle 2 blieb die Probe B1 bei Lagerung bei 40°C am längsten homogen und klar bzw. zeigte den geringsten pH-Wertabfall und somit die beste Stabilität gegen Hydrolyse.

Die Lagerstabilität war deutlich verbessert im Vergleich zur ungepufferten Probe (Vergleichsbeispiel VB1).

Die Puffersubstanzen alleine (Vergleichsbeispiele VB2 und VB3) bzw. in anderem Verhältnis kombiniert (Vergleichsbeispiel VB4) zeigten ein signifikant schlechteres Ergebnis als B1.

### Beispiel B2:

Das Alkyllactatsulfat ALS-II wurde mit 0,5% Kaliumdihydrogenphosphat und 1,0% Natriumcitrat versetzt und dann bei 20 °C gerührt bis alle Feststoffe gelöst waren und eine klare homogene Lösung vorlag. Aniontensidgehalt, pH-Wert und Aussehen des Musters während der Lagerung wurden bestimmt. Aus Tabelle 3 ist zu erkennen, dass die Abnahme des pH-Werts und das Aussehen der Probe mit einer Abnahme des Aniontensidgehalts korrelieren.

**Tabelle 3: Aniontensidgehalt, pH-Wert und Aussehen des Beispielmusters B2 während Lagerung bei 40°C**

| | Start | 1. Monat | 2. Monat | 3. Monat | 4. Monat | 5. Monat | 6. Monat | 7. Monat |
|---|---|---|---|---|---|---|---|---|
| Aniontensid [Gew.-%] | 14,7 | 14,3 | 13,9 | 13,5 | 13,3 | 12,9 | 12,5 | 11,7 |
| pH-Wert | 6,70 | 6,14 | 5,97 | 5,89 | 5,34 | 5,06 | 4,70 | 4,31 |
| Aussehen | klar | klar | klar | klar | klar | leichte Schlieren, nach Schütteln wieder klar | milchig trüb | 2-phasig |

## Patentansprüche

1. Zusammensetzung enthaltend
• ein oder mehrere **Alkyllactatsulfate (A)** der allgemeinen Formel (I),
R¹(OCOCH(CH₃))ₙOSO₃M¹ (I)
worin der Rest R¹ einen linearen oder verzweigten Alkyl- oder Alkenylrest mit 6 bis 30 C-Atomen bedeutet, der Index n eine Zahl im Bereich von 1 bis 5 ist, und der Rest M¹ ausgewählt wird aus der Gruppe H, Li, Na, K, Ca/2, Mg/2, Ammonium und Alkanolamin,
• ein oder mehrere **Verbindungen (B),** die ausgewählt werden aus der Gruppe Phosphorsäure und deren Mono-, Di-, und Trisalzen, wobei das Kation dieser Salze ausgewählt wird aus der Gruppe Li, Na, K, Ca, Mg, Ammonium und Alkanolamin,
• ein oder mehrere **Verbindungen (C),** die ausgewählt werden aus der Gruppe Citronensäure und deren Mono-, Di-, und Trisalzen, wobei das Kation dieser Salze ausgewählt wird aus der Gruppe Li, Na, K, Ca, Mg, Ammonium und Alkanolamin, und
• **Wasser**
wobei folgende Maßgaben gelten:
• Das Gewichtsverhältnis der Summe der Verbindungen (B) und (C) zur Summe der Verbindungen (A) liegt im Bereich von 1 : 5 bis 1 : 10;
• das Gewichtsverhältnis der Summe der Verbindungen (B) zur Summe der Verbindungen (C) liegt im Bereich von 1 : 1,2 bis 1 : 3;
• der pH-Wert der Zusammensetzungen liegt im Bereich von 4,5 bis 7,5.

2. Zusammensetzungen nach Anspruch 1, wobei der Rest R¹ in der Formel (I) ein gesättigter linearer Alkylrest mit 12 bis 16 C-Atomen ist.

3. Zusammensetzungen nach Anspruch 1 oder 2, wobei der Rest M¹ Natrium (Na) oder Kalium (K) bedeutet.

4. Zusammensetzungen nach einem der Ansprüche 1 bis 3, wobei die Verbindungen (A) in einer Menge im Bereich von 5 bis 50 Gew.-% - bezogen auf die gesamte Zusammensetzung - vorhanden sind.

5. Zusammensetzungen nach einem der Ansprüche 1 bis 4, wobei der pH-Wert der Zusammensetzung im Bereich von 5,0 bis 7,0 liegt.

6. Zusammensetzungen nach einem der Ansprüche 1 bis 5, wobei das Gewichtsverhältnis der Summe der Verbindungen (B) und (C) zu den Verbindungen (A) im Bereich von 1 : 6 bis 1 : 7,5 liegt.

7. Zusammensetzungen nach einem der Ansprüche 1 bis 6, wobei das Gewichtsverhältnis der Verbindungen (B) zu den Verbindungen (C) im Bereich von 1 : 1,2 bis 1 : 3 liegt.

8. Verwendung der Zusammensetzungen nach einem der Ansprüche 1 bis 7 für kosmetische Mittel sowie Wasch- und Reinigungsmittel.

9. Verwendung der Zusammensetzungen nach einem der Ansprüche 1 bis 7 für kosmetische Mittel in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen, Rasiercremes und Zahnpflegeprodukten.

10. Verwendung der Zusammensetzungen nach einem der Ansprüche 1 bis 7 für Mittel mit niedrigem pH-Wert zur Reinigung harter Oberflächen, wie Bad- und WC-Reiniger und dergleichen, sowie für Reinigungs- und/oder Duftgele zur Anwendung in sanitären Einrichtungen.
